# EUROPEAN PATENT APPLICATION

(11) **EP 4 424 693 A1**
(43) Date of publication of application: **04.09.2024**
(21) Application number: 23892745.3
(22) Date of filing: 25.05.2023
(51) Int. Cl.: C07H 3/02, C07H 1/06

(54) **PREPARATION METHOD FOR HIGH-PURITY ARABINOSE CRYSTAL**

(30) Priority: 17.12.2022 CN 202211628377
(71) Applicant: Zhejiang Huakang Pharmaceutical Co., Ltd., Quzhou, Zhejiang 324302 (CN)
(72) Inventor: HAN, Xinfeng, Quzhou, Zhejiang 324302 (CN); LI, Dongxu, Quzhou, Zhejiang 324302 (CN); YANG, Minqian, Quzhou, Zhejiang 324302 (CN); LIAO, Chengjun, Quzhou, Zhejiang 324302 (CN); QIN, Shufang, Quzhou, Zhejiang 324302 (CN); LUO, Jiaxing, Quzhou, Zhejiang 324302 (CN)
(74) Representative: Sun, Yiming
(86) International application number: PCT/CN2023/096370
(87) International publication number: WO 2024/124812

(57) **Abstract**

The present disclosure relates to a method for preparing a high-purity arabinose crystal, comprising operations of dissolving, blending, ion exchange, decolorization and filtration, fine filtration, evaporation and concentration, crystallization, centrifugation, and drying performed in sequence on a low-purity arabinose crystal. Throughout the operations of preparing the arabinose crystal, a pH value of a material is controlled between 4.3 and 7.5, and a temperature does not exceed 70°C. This prevents arabinose from transforming into impurities under a high temperature, thereby maintaining and improving a purity of the prepared arabinose crystal.

## Description

### TECHNICAL FIELD

The present disclosure relates to the field of sugar alcohol preparation technology, and in particular to a method for preparing a high-purity arabinose crystal.

### BACKGROUND

Arabinose is widely used in fields such as pharmaceuticals and health foods. A low-purity arabinose crystal product is inexpensive but fails to meet customer demands. On the other hand, a high-purity arabinose crystal product, due to its complex processes and production difficulties, commands higher prices but can meet specific customer requirements for process formulations, thereby possessing a significant market space.

In an existing production process, L-arabinose, due to its characteristics, may be transformed into other substances by reaction under a high temperature and a low pH value. For example, the L-arabinose may be transformed into xylose by isomerization. As another example, the L-arabinose may break down into small molecules below five carbons by decomposition. As yet another example, the L-arabinose may transform into maltotriose by polymerization. Consequently, the arabinose content in the L-arabinose decreases continuously in the production process, particularly during operations like decolorization, evaporation, and chromatographic separation, making it challenging to obtain a high-purity arabinose crystal with purity above 99.5%.

CN112079886A discloses a method for improving the purity of xylose and arabinose through chromatographic separation. However, the method does not consider the issue of arabinose transformation due to a relatively high temperature and a relatively low pH value in the production operations such as decolorization and chromatographic separation.

### SUMMARY

The technical problem addressed by the present disclosure is to provide a method for preparing a high-purity arabinose crystal. This is achieved by controlling a pH value and a temperature during preparation to prevent isomerization, decomposition, and polymerization, thereby enhancing the purity of the arabinose crystal.

The method for preparing the high-purity arabinose crystal includes:
Operation 1, dissolution: dissolving a low-purity arabinose crystal to obtain a dissolved sugar solution using a dissolution tank system, wherein the dissolution tank system is provided with a temperature sensor and an automatic regulating valve, a Siemens distributed control system (DCS) is used to monitor a temperature during the dissolution to ensure that a temperature of the dissolved sugar solution is in a range of 55°C-60°C, and an arabinose content in the low-purity arabinose crystal is 96%-97%.
Operation 2, blending: directing the dissolved sugar solution through a pipeline into a blending tank using an electromagnetic flow meter, an automatic regulating valve, and a Siemens intelligent control system, adding an arabinose centrifugation mother liquor from operation 8 to the blending tank, and obtaining a blended sugar solution by blending the dissolved sugar solution with the arabinose centrifugation mother liquor, wherein a pH value of the blended sugar solution is in a range of 4.3-5.0, a dry basis concentration is a range of 50%-60%, and the arabinose content of the blended sugar solution is 94±0.5%.
Operation 3, ion exchange: performing an ion exchange operation on the blended sugar solution to obtain an ion-exchanged sugar solution, wherein a material temperature is controlled at 45°C -50°C through a heat exchanger for ion exchange feed, and a pH value of the ion-exchanged sugar solution is in a range of 5.5-6.5.
Operation 4, decolorization and filtration: directing the ion-exchanged sugar solution through a pipeline into a decolorization tank for decolorization and filtration to obtain a decolorized sugar solution, wherein a decolorization temperature for decolorization and filtration is controlled at 60°C-65°C, and a pH value for decolorization and filtration is in a range of 5.5-7.5.
Operation 5, fine filtration: performing a fine filtration operation on the decolorized sugar solution using a fine filtration membrane with an aperture of 0.45µm to obtain a refined sugar solution, wherein a temperature for fine filtration is controlled at 50°C-65°C, and a pH value for fine filtration is in a range of 5.0-7.5.
Operation 6, evaporation and concentration: directing the refined sugar solution into a mechanical vapor recompression (MVR) evaporator for evaporation and concentration to obtain a concentrated sugar solution, wherein a temperature for evaporation and concentration is at 65°C-70°C, and a pH value for evaporation and concentration is in a range of 5.0-7.5.
Operation 7, crystallization: introducing the concentrated sugar solution into a vacuum sugar-cooking system for crystallization, wherein a temperature for crystallization is at 63°C-65°C, and a vacuum level for crystallization is in a range of 70 mbar-90 mbar.
Operation 8, centrifugation: performing a centrifugal separation operation on a material obtained in operation 7 to separate a solid arabinose and an arabinose centrifugation mother liquor from the material, wherein the arabinose centrifugation mother liquor is directed through the pipeline into the blending tank for blending in operation 2 and an arabinose content in the arabinose centrifugation mother liquor is in a range of 89%-91%.
Operation 9, drying: performing a drying operation on the solid arabinose with 80°C hot air to obtain the high-purity arabinose crystal, wherein an arabinose content of the high-purity arabinose crystal is greater than 99.8%.

The method strictly controls the temperatures during the preparation operations, ensuring that the temperatures do not exceed 70°C, thereby preventing arabinose from transforming into impurities under a high temperature and reducing purity.

Compared to the existing technology, the method for preparing the high-purity arabinose crystal in the present disclosure has the following beneficial effects:
(1) The blending operation introduced before refinement ensures the stability of the liquid material.
(2) By adjusting the ratio of cationic resins to anionic resins to 7:10, the pH value of the ion-exchanged output (i.e., the ion-exchanged sugar solution) is stabilized at 5.5-6.5. This avoids local overalkalinization of the liquid material during pH adjustment by adding alkalis, preventing isomerization issue and maintaining production efficiency and product quality in subsequent operations.
(3) Using the low-purity arabinose crystal with a relatively higher impurity content, the method performs dissolution, purification, and concentration operations on the low-purity arabinose crystal to obtain the arabinose crystal with a purity greater than 99.8%. This results in a crystallization yield improvement of more than 3.5% compared to existing production processes.

### BRIEF DESCRIPTION OF THE DRAWINGS

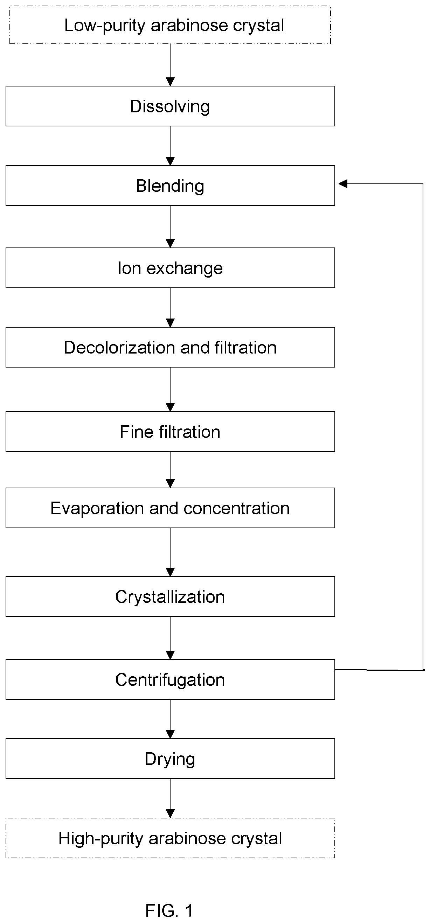

FIG. 1 is a flowchart illustrating an exemplary process for preparing a high-purity arabinose crystal according to some embodiments of the present disclosure.

### DETAILED DESCRIPTION

In order to more clearly illustrate the technical solutions and beneficial effects of the embodiments of the present disclosure, the accompanying drawings for the description of the embodiments are described below. It should be understood that the specific embodiments described herein are only intended to explain the present disclosure and are not intended to limit the scope of the present disclosure.

Please refer to FIG. 1 for a preferred embodiment of a method for preparing a high-purity arabinose crystal according to the present disclosure. The directional arrows in the drawing indicate the flow direction of materials or the sequence of the operations. The method includes:
Operation 1, dissolution: a low-purity arabinose crystal is dissolved to obtain a dissolved sugar solution using a dissolution tank system. The dissolution tank system is provided with a temperature sensor and an automatic regulating valve, a Siemens distributed control system (DCS) is used to monitor a temperature during the dissolution to ensure that a temperature of the dissolved sugar solution is in a range of 55°C-60°C, and an arabinose content in the low-purity arabinose crystal is 96%-97%, thereby ensuring a stability of the dissolved sugar solution.
Operation 2, blending: the dissolved sugar solution is directed into a blending tank through a pipeline using an electromagnetic flow meter, an automatic regulating valve, and a Siemens intelligent control system, an arabinose centrifugation mother liquor from operation 8 is added to the blending tank, and a blended sugar solution is obtained by blending the dissolved sugar solution with the arabinose centrifugation mother liquor. The pH value of the blended sugar solution is in a range of 4.3-5.0, a dry basis concentration is in a range of 50%-60%, and the arabinose content of the blended sugar solution is 94±0.5%.
Operation 3, ion exchange: an ion exchange operation is performed on the blended sugar solution to obtain an ion-exchanged sugar solution. The material temperature is controlled at 45°C -50°C through a heat exchanger for ion exchange feed, and the pH value of the ion-exchanged sugar solution is in a range of 5.5-6.5. An ion exchange column is used in the ion exchange operation, and a ratio of cationic resins to anionic resins in the ion exchange column is 7:10. By adjusting the ratio of cationic resins to anionic resins to 7:10, the method maintains the pH value of the ion-exchanged sugar solution in the range of 5.5-6.5, thereby preventing the arabinose from being transformed into impurities under an excessively low pH value.
Operation 4, decolorization and filtration: the ion-exchanged sugar solution is directed through a pipeline into a decolorization tank for decolorization and filtration to obtain a decolorized sugar solution, wherein a decolorization temperature for decolorization and filtration is controlled at 60°C-65°C, and a pH value for decolorization and filtration is in a range of 5.5-7.5. In the decolorization filtration operation, activated carbon is added to the decolorization tank at a ratio of 1.2-1.4 Kg/ton of the dry basis and stirred at 110rpm for 30-45 minutes for decolorization, and a plate and frame filter is used to filter and remove the activated carbon.
Operation 5, fine filtration: a fine filtration operation is performed on the decolorized sugar solution using a fine filtration membrane with an aperture of 0.45µm to obtain a refined sugar solution. The temperature for fine filtration is controlled at 50°C-65°C and the pH value for fine filtration is in a range of 5.0-7.5.
Operation 6, evaporation and concentration: the refined sugar solution is directed into a mechanical vapor recompression (MVR) evaporator for evaporation and concentration to obtain a concentrated sugar solution. The temperature for evaporation and concentration is in a range of 65°C-70°C and the pH value for evaporation and concentration is in a range of 5.0-7.5.
Operation 7, crystallization: the concentrated sugar solution may be introduced into a vacuum sugar-cooking system for crystallization. The temperature for crystallization is in a range of 63°C-65°C, and the vacuum level for crystallization is in a range of 70 mbar-90 mbar. In the crystallization operation, arabinose crystal seeds of 300-400 mesh in a ratio of two per ten thousand of the dry basis are added when the oversaturation degree of the concentrated sugar solution reaches 1.01-1.02. A crystallization period is 8 hours, and a stirring speed is at 80rpm.
Operation 8, centrifugation: a centrifugal separation operation is performed on a material obtained in operation 7 to separate a solid arabinose and an arabinose centrifugation mother liquor from the material. The arabinose centrifugation mother liquor is directed through the pipeline into the blending tank for blending in operation 2 and an arabinose content in the arabinose centrifugation mother liquor is in a range of 89%-91%. In the centrifugal separation operation, a washing time is controlled at 10 seconds and a water temperature is maintained at 55°C-60°C.
Operation 9, drying: a drying operation is performed on the solid arabinose with 80°C hot air to obtain the high-purity arabinose crystal, and an arabinose content of the high-purity arabinose crystal is greater than 99.8%. In the drying operation, a moisture content is controlled in a range of 0.15%-0.3%, and after the moisture content is in the range of 0.15%-0.3%, the high-purity arabinose crystal is cooled to 20°C - 24°C using clean cold air with a temperature in a range of 12°C-15°C.

The method strictly controls the temperature during the preparation operations, ensuring that the temperature does not exceed 70°C, thereby preventing arabinose from being transformed into impurities under a high temperature and reducing purity.

The method for preparing the high-purity arabinose crystal in the present disclosure is further illustrated through the following embodiments.

### Embodiment 1: Determining an impact of temperature and pH value on arabinose content

### Embodiment 1 includes the following operations:

100g of L-arabinose sample was taken and dissolved in water to form an arabinose solution with a refractive index of 60%. The pH value of the arabinose solution was adjusted to 2.7 and 4.3. The arabinose solution was sequentially heated at 60°C, 65°C, and 70°C for 48 hours, respectively. Samples were collected at 24 hours and 48 hours for analysis and results are shown in Table 1.

**Table 1. Components of the arabinose solution under different temperatures and pH values**

| Components | Content (%) | | | | | | | | | | | | |
|---|---|---|---|---|---|---|---|---|---|---|---|---|---|
| | Initial | pH2.7 | | | | | | pH4.3 | | | | | |
| | | 60°C | | 65°C | | 70°C | | 60°C | | 65°C | | 70°C | |
| | | 24h | 48h | 24h | 48h | 24h | 48h | 24h | 48h | 24h | 48h | 24h | 48h |
| Arabinose | 98.34 | 97.6 | 96.75 | 97.33 | 95.96 | 96.83 | 95.4 | 98.3 | 98.11 | 97.97 | 97.89 | 98.06 | 97.54 |
| Xylose | 1.36 | 1.55 | 1.78 | 1.88 | 2.09 | 1.9 | 2.23 | 1.42 | 1.61 | 1.71 | 1.75 | 1.68 | 1.84 |
| Maltotriose | 0.05 | 0.32 | 0.55 | 0.4 | 0.93 | 0.6 | 1.17 | 0.08 | 0.15 | 0.2 | 0.23 | 0.26 | 0.32 |
| Glucose/ Sorbitol | 0 | 0.12 | 0.29 | 0.11 | 0.19 | 0.11 | 0.13 | 0 | 0 | 0 | 0.06 | 0 | 0.08 |

Comparing the results, as the pH value decreases and the heating temperature increases during arabinose production, the arabinose content decreases more rapidly over time. The pH value is identified as a main factor causing a decline in the arabinose content. When the pH value is at 4.3 or above, and the material is heated at 75°C for 48 hours, the arabinose content decreases only by 2.31%. However, when the pH value is at 2.7, and the material is heated at 75°C for 48 hours, the arabinose content decreases by 8.9%, which is more than three times the decrease of the arabinose content when the pH value is at 4.3.

The results indicate that effectively adjusting the pH value of the arabinose solution to above 4.3 can reduce the impact of high temperature on the arabinose content, avoiding to lower the processing temperature for content control, thereby maintaining production efficiency.

### Embodiment 2:

A first embodiment of the method for preparing the high-purity arabinose crystal in the present disclosure includes:
Operation 11, dissolution: a purchased low-purity arabinose crystal was dissolved to obtain a dissolved sugar solution using a dissolution tank system. The dissolution tank system was provided with a temperature sensor and an automatic regulating valve, a Siemens DCS was used to monitor a temperature during the dissolution to ensure that a temperature of the dissolved sugar solution was in a range of 55°C-60°C, and the arabinose content in the low-purity arabinose crystal was 96%.
Operation 12, blending: the dissolved sugar solution obtained in operation 11 was directed through a pipeline into a blending tank using an electromagnetic flowmeter, an automatic regulating valve, and a Siemens intelligent control system. An arabinose centrifugation mother liquor from operation 18 was added to the blending tank, and a blended sugar solution was obtained by blending the dissolved sugar solution with the arabinose centrifugation mother liquor. The pH value of the blended sugar solution was 5.0, a dry basis concentration was 50%, and the arabinose content of the blended sugar solution was 93.84%.
Operation 13, ion exchange: an ion exchange operation was performed on the blended sugar solution obtained in operation 12 to obtain an ion-exchanged sugar solution using an ion exchange system, wherein the material temperature was controlled at 45°C -50°C through a heat exchanger for ion exchange feed, a ratio of cationic resins to anionic resins of the ion exchange system was adjusted to be 7:10, and the pH value of the ion-exchanged sugar solution was 6.5.
Operation 14, decolorization and filtration: the ion-exchanged sugar solution was directed through a pipeline into a decolorization tank, activated carbon was added to the decolorization tank at a ratio of 1.2 Kg/ton of the dry basis and stirred at 110rpm for 30 minutes for decolorization, and a plate and frame filter was used to filter and remove the activated carbon to obtain a decolorized sugar solution. The decolorization temperature for decolorization and filtration was controlled at 60°C, and the pH value for decolorization and filtration was in a range of 5.0-7.5.
Operation 15, fine filtration: a fine filtration operation was performed on the decolorized sugar solution obtained in operation 14 using a fine filtration membrane with an aperture of 0.45µm to obtain a refined sugar solution. The temperature for fine filtration was controlled at 50°C-65°C, and the pH value for fine filtration was in a range of 5.0-7.5.
Operation 16, evaporation and concentration: the refined sugar solution obtained in operation 15 was directed into an MVR evaporator for evaporation and concentration to obtain a concentrated sugar solution. The temperature for evaporation and concentration was at 68°C, and the pH value for evaporation and concentration was in a range of 5.0-7.5.
Operation 17, crystallization: the concentrated sugar solution obtained in operation 16 was introduced into a vacuum sugar-cooking system for crystallization. The temperature for crystallization was at 63°C-65°C, and the vacuum level for crystallization was in a range of 70 mbar-90 mbar. Arabinose crystal seeds of 300-400 mesh were added in a ratio of two per ten thousand of the dry basis when an oversaturation degree of the concentrated sugar solution reached 1.01-1.02. A crystallization period was 8 hours, and a stirring speed was at 80rpm.
Operation 18, centrifugation: a centrifugal separation operation was performed on a material obtained in operation 17 using a centrifuge machine, wherein a washing time was controlled at 10 seconds and a water temperature was maintained at 55°C-60°C. A solid arabinose and an arabinose centrifugation mother liquor were separate from the material. The solid arabinose was dried in operation 19, the arabinose centrifugation mother liquor was directed through the pipeline into the blending tank for blending in operation 12, and the arabinose content in the arabinose centrifugation mother liquor was 89%.
Operation 19, drying and packaging: a drying operation was performed on the solid arabinose obtained in operation 18 with 80°C hot air to obtain a high-purity arabinose crystal, wherein a moisture content was controlled in a range of 0.15%-0.3%, and after the moisture content was in the range of 0.15%-0.3%, the high-purity arabinose crystal was cooled to 20°C -24°C using clean cold air at 12°C-15°C, and the arabinose content of the high-purity arabinose crystal is greater than 99.8%. The high-purity arabinose crystal obtained after drying was packaged using a packaging machine.

### Embodiment 3:

A second embodiment of the method for preparing the high-purity arabinose crystal in the present disclosure includes:
Operation 21, dissolution: a purchased low-purity arabinose crystal was dissolved to obtain a dissolved sugar solution using a dissolution tank system. The dissolution tank system was provided with a temperature sensor and an automatic regulating valve, a Siemens DCS was used to monitor a temperature during the dissolution to ensure that a temperature of the dissolved sugar solution was in a range of 55°C-60°C, and the arabinose content in the low-purity arabinose crystal was 97%.
Operation 22, blending: the dissolved sugar solution obtained in operation 21 was directed through a pipeline into a blending tank using an electromagnetic flowmeter, an automatic regulating valve, and a Siemens intelligent control system. An arabinose centrifugation mother liquor from operation 28 was added to the blending tank, and a blended sugar solution was obtained by blending the dissolved sugar solution with the arabinose centrifugation mother liquor. The pH value of the blended sugar solution was in a range of 4.3-5.0, a dry basis concentration was 60%, and the arabinose content of the blended sugar solution was 94.57%.
Operation 23, ion exchange: an ion exchange operation was performed on the blended sugar solution obtained in operation 22 to obtain an ion-exchanged sugar solution using an ion exchange system. The temperature of the sugar solution was controlled at 45°C -50°C through a heat exchanger for ion exchange feed, a ratio of cationic resins to anionic resins of the ion exchange system was adjusted to be 7:10, and the pH value of the ion-exchanged sugar solution was 6.0.
Operation 24, decolorization and filtration: the ion-exchanged sugar solution was directed through a pipeline into a decolorization tank, activated carbon was added to the decolorization tank at a ratio of 1.4 Kg/ton of the dry basis and stirred at 110rpm for 35 minutes for decolorization, and a plate and frame filter was used to filter and remove the activated carbon to obtain a decolorized sugar solution. The decolorization temperature for decolorization and filtration was controlled at 62°C, and the pH value for decolorization and filtration was in a range of 5.5-7.5.
Operation 25, fine filtration: a fine filtration operation was performed on the decolorized sugar solution obtained in operation 24 using a fine filtration membrane with an aperture of 0.45µm to obtain a refined sugar solution. The temperature for fine filtration was controlled at 50°C-65°C, and the pH value for fine filtration was in a range of 5.0-7.5.
Operation 26, evaporation and concentration: the refined sugar solution obtained in operation 25 was directed into an MVR evaporator for evaporation and concentration to obtain a concentrated sugar solution. The temperature for evaporation and concentration was at 68°C, and the pH value for evaporation and concentration was in a range of 5.0-7.5.
Operation 27, crystallization: the concentrated sugar solution obtained in operation 26 was introduced into a vacuum sugar-cooking system for crystallization. The temperature for crystallization was at 63°C-65°C, and the vacuum level for crystallization was in a range of 70 mbar-90 mbar. Arabinose crystal seeds of 300-400 mesh were added in a ratio of two per ten thousand of the dry basis when an oversaturation degree of the concentrated sugar solution reached 1.01-1.02. A crystallization period was 8 hours, and a stirring speed was at 80rpm.
Operation 28, centrifugation: a centrifugal separation operation was performed on a material obtained in operation 27 using a centrifuge machine, wherein a washing time was controlled at 10 seconds and a water temperature was maintained at 55°C-60°C. A solid arabinose and an arabinose centrifugation mother liquor were separate from the material. The solid arabinose was dried in operation 29, the arabinose centrifugation mother liquor was directed through the pipeline into the blending tank for blending in operation 22, and the arabinose content in the arabinose centrifugation mother liquor was 91%.
Operation 29, drying and packaging: a drying operation was performed on the solid arabinose obtained in operation 28 with 80°C hot air to obtain a high-purity arabinose crystal, wherein a moisture content was controlled in a range of 0.15%-0.3%, and after the moisture content was in the range of 0.15%-0.3%, the high-purity arabinose crystal was cooled to 20°C-24°C using clean cold air at 12°C-15°C, and the arabinose content of the high-purity arabinose crystal is greater than 99.8%. The high-purity arabinose crystal obtained after drying was packaged using a packaging machine.

### Embodiment 4:

A third embodiment of the method for preparing the high-purity arabinose crystal in the present disclosure includes:
Operation 31, dissolution: a purchased low-purity arabinose crystal was dissolved to obtain a dissolved sugar solution using a dissolution tank system. The dissolution tank system was provided with a temperature sensor and an automatic regulating valve, a Siemens DCS was used to monitor a temperature during the dissolution to ensure that a temperature of the dissolved sugar solution was in a range of 55°C-60°C, and the arabinose content in the low-purity arabinose crystal was 96.5%.
Operation 32, blending: the dissolved sugar solution obtained in operation 31 was directed through a pipeline into a blending tank using an electromagnetic flowmeter, an automatic regulating valve, and a Siemens intelligent control system. An arabinose centrifugation mother liquor from operation 38 was added to the blending tank, and a blended sugar solution was obtained by blending the dissolved sugar solution with the arabinose centrifugation mother liquor. The pH value of the blended sugar solution was in a range of 4.3-5.0, a dry basis concentration was 55%, and the arabinose content of the blended sugar solution was 94.50%.
Operation 33, ion exchange: an ion exchange operation was performed on the blended sugar solution obtained in operation 32 to obtain an ion-exchanged sugar solution using an ion exchange system. The temperature of the sugar solution was controlled at 45°C -50°C through a heat exchanger for ion exchange feed, a ratio of cationic resins to anionic resins of the ion exchange system was adjusted to be 7:10, and the pH value of the ion-exchanged sugar solution was 5.5.
Operation 34, decolorization and filtration: the ion-exchanged sugar solution was directed through a pipeline into a decolorization tank, activated carbon was added to the decolorization tank at a ratio of 1.25 Kg/ton of the dry basis and stirred at 110rpm for 45 minutes for decolorization, and a plate and frame filter was used to filter and remove the activated carbon to obtain a decolorized sugar solution. The decolorization temperature for decolorization and filtration was controlled at 65°C, and the pH value for decolorization and filtration was in a range of 5.5-7.5.
Operation 35, fine filtration: a fine filtration operation was performed on the decolorized sugar solution obtained in operation 34 using a fine filtration membrane with an aperture of 0.45µm to obtain a refined sugar solution. The temperature for fine filtration was controlled at 50°C-65°C, and the pH value for fine filtration was in a range of 5.0-7.5.
Operation 36, evaporation and concentration: the refined sugar solution obtained in operation 35 was directed into an MVR evaporator for evaporation and concentration to obtain a concentrated sugar solution. The temperature for evaporation and concentration was at 70°C, and the pH value for evaporation and concentration was in a range of 5.0-7.5.
Operation 37, crystallization: the concentrated sugar solution obtained in operation 36 was introduced into a vacuum sugar-cooking system for crystallization. The temperature for crystallization was at 63°C-65°C, and the vacuum level for crystallization was in a range of 70 mbar-90 mbar. Arabinose crystal seeds of 300-400 mesh were added in a ratio of two per ten thousand of the dry basis when an oversaturation degree of the concentrated sugar solution reached 1.01-1.02. A crystallization period was 8 hours, and a stirring speed was at 80rpm.
Operation 38, centrifugation: a centrifugal separation operation was performed on a material obtained in operation 37 using a centrifuge machine, wherein a washing time was controlled at 10 seconds and a water temperature was maintained at 55°C-60°C. A solid arabinose and an arabinose centrifugation mother liquor were separate from the material. The solid arabinose was dried in operation 39, the arabinose centrifugation mother liquor was directed through the pipeline into the blending tank for blending in operation 32, and the arabinose content in the arabinose centrifugation mother liquor was 90.3%.
Operation 39, drying and packaging: a drying operation was performed on the solid arabinose obtained in operation 38 with 80°C hot air to obtain a high-purity arabinose crystal, wherein a moisture content was controlled in a range of 0.15%-0.3%, and after the moisture content was in the range of 0.15%-0.3%, the high-purity arabinose crystal was cooled to 20°C-24°C using clean cold air at 12°C-15°C, and the arabinose content of the high-purity arabinose crystal is greater than 99.8%. The high-purity arabinose crystal obtained after drying was packaged using a packaging machine.

### Embodiment 5:

A fourth embodiment of the method for preparing the high-purity arabinose crystal in the present disclosure includes:
Operation 41, dissolution: a purchased low-purity arabinose crystal was dissolved to obtain a dissolved sugar solution using a dissolution tank system. The dissolution tank system was provided with a temperature sensor and an automatic regulating valve, a Siemens DCS was used to monitor a temperature during the dissolution to ensure that a temperature of the dissolved sugar solution was in a range of 55°C-60°C, and the arabinose content in the low-purity arabinose crystal was 96.9%.
Operation 42, blending: the dissolved sugar solution obtained in operation 41 was directed through a pipeline into a blending tank using an electromagnetic flowmeter, an automatic regulating valve, and a Siemens intelligent control system. An arabinose centrifugation mother liquor from operation 48 was added to the blending tank, and a blended sugar solution was obtained by blending the dissolved sugar solution with the arabinose centrifugation mother liquor. The pH value of the blended sugar solution was in a range of 4.3-5.0, a dry basis concentration was 60%, and the arabinose content of the blended sugar solution was 94.43%.
Operation 43, ion exchange: an ion exchange operation was performed on the blended sugar solution obtained in operation 42 to obtain an ion-exchanged sugar solution using an ion exchange system, wherein the temperature of the sugar solution was controlled at 45°C -50°C through a heat exchanger for ion exchange feed, a ratio of cationic resins to anionic resins of the ion exchange system was adjusted to be 7:10, and the pH value of the ion-exchanged sugar solution was 6.2.
Operation 44, decolorization and filtration: the ion-exchanged sugar solution was directed through a pipeline into a decolorization tank, activated carbon was added to the decolorization tank at a ratio of 1.38 Kg/ton of the dry basis and stirred at 110rpm for 40 minutes for decolorization, and a plate and frame filter was used to filter and remove the activated carbon to obtain a decolorized sugar solution. The decolorization temperature for decolorization and filtration was controlled at 60°C, and the pH value for decolorization and filtration was in a range of 5.5-7.5.
Operation 45, fine filtration: a fine filtration operation was performed on the decolorized sugar solution obtained in operation 44 using a fine filtration membrane with an aperture of 0.45µm to obtain a refined sugar solution. The temperature for fine filtration was controlled at 50°C-65°C, and the pH value for fine filtration was in a range of 5.0-7.5.
Operation 46, evaporation and concentration: the refined sugar solution obtained in operation 45 was directed into an MVR evaporator for evaporation and concentration to obtain a concentrated sugar solution. The temperature for evaporation and concentration was at 65°C, and the pH value for evaporation and concentration was in a range of 5.0-7.5.
Operation 47, crystallization: the concentrated sugar solution obtained in operation 46 was introduced into a vacuum sugar-cooking system for crystallization, wherein a temperature for crystallization was at 63°C-65°C, and a vacuum level for crystallization was in a range of 70 mbar-90 mbar. Arabinose crystal seeds of 300-400 mesh were added in a ratio of two per ten thousand of the dry basis when an oversaturation degree of the concentrated sugar solution reached 1.01-1.02. A crystallization period was 8 hours, and a stirring speed was at 80rpm.
Operation 48, centrifugation: a centrifugal separation operation was performed on a material obtained in operation 47 using a centrifuge machine, wherein a washing time was controlled at 10 seconds and a water temperature was maintained at 55°C-60°C. A solid arabinose and an arabinose centrifugation mother liquor were separate from the material. The solid arabinose was dried in operation 49, the arabinose centrifugation mother liquor was directed through the pipeline into the blending tank for blending in operation 42, and the arabinose content in the arabinose centrifugation mother liquor was 89.8%.
Operation 49, drying and packaging: a drying operation was performed on the solid arabinose obtained in operation 48 with 80°C hot air to obtain a high-purity arabinose crystal, wherein a moisture content was controlled in a range of 0.15%-0.3%, and after the moisture content was in the range of 0.15%-0.3%, the high-purity arabinose crystal was cooled to 20°C-24°C using clean cold air at 12°C-15°C, and the arabinose content of the high-purity arabinose crystal is greater than 99.8%. The high-purity arabinose crystal obtained after drying was packaged using a packaging machine.

The technical effects of the method of preparing the high-purity arabinose crystal in the present disclosure are further illustrated below through a control group.

### Control group

Conventional preparation operations for preparing an arabinose crystal were adopted for the control group, and a ratio of cationic resins to anionic resins was 1:1.

Operation 51, dissolution: a purchased low-purity arabinose crystal was dissolved to obtain a dissolved sugar solution using a dissolution tank system. The dissolution tank system was provided with a temperature sensor and an automatic regulating valve, a Siemens DCS was used to monitor a temperature during the dissolution to ensure that a temperature of the dissolved sugar solution was in a range of 55°C-60°C, the arabinose content in the low-purity arabinose crystal was 97%, a dry basis concentration was 55%, and the pH value of the dissolved sugar solution was in a range of 3.5-5.0.

Operation 52, ion exchange: an ion exchange operation was performed on the dissolved sugar solution obtained in operation 51 to obtain an ion-exchanged sugar solution using an ion exchange system, wherein the temperature of the sugar solution was controlled at 45°C -50°C through a heat exchanger for ion exchange feed, a ratio of cationic resins to anionic resins of the ion exchange system was adjusted to be 1:1, and the pH value of the ion-exchanged sugar solution was in a range of 3.5-4.0.

Operation 53, decolorization and filtration: the ion-exchanged sugar solution was directed through a pipeline into a decolorization tank, activated carbon was added to the decolorization tank at a ratio of 1.4 Kg/ton of the dry basis and stirred at 110rpm for 45 minutes for decolorization, and a plate and frame filter was used to filter and remove the activated carbon to obtain a decolorized sugar solution. The decolorization temperature for decolorization and filtration was controlled at 75°C-80°C, and a pH value for decolorization and filtration was in a range of 3.5-4.5.

Operation 54, fine filtration: a fine filtration operation was performed on the decolorized sugar solution obtained in operation 53 using a fine filtration membrane with an aperture of 0.45µm to obtain a refined sugar solution. The temperature for fine filtration was controlled at 50°C-65°C, and the pH value for fine filtration was in a range of 3.5-4.5.

Operation 55, evaporation and concentration: the refined sugar solution obtained in operation 54 was directed into a falling film evaporator for evaporation and concentration to obtain a concentrated sugar solution. The temperature for evaporation and concentration was in a arrange of 65°C-98°C, and the pH value for evaporation and concentration was in a range of 3.5-4.5.

Operation 56, crystallization: the concentrated sugar solution obtained in operation 55 was introduced into a vacuum sugar-cooking system for crystallization. The temperature for crystallization was at 63°C-65°C, and the vacuum level for crystallization was in a range of 70 mbar-90 mbar. Arabinose crystal seeds of 300-400 mesh were added in a ratio of two per ten thousand of the dry basis when an oversaturation degree of the concentrated sugar solution reached 1.01-1.02. A crystallization period was 8 hours, and a stirring speed was at 80rpm.

Operation 57, centrifugation: a centrifugal separation operation was performed on a material obtained in operation 56 using a centrifuge machine, wherein a washing time was controlled at 10 seconds and a water temperature was maintained at 55°C-60°C. A solid arabinose and an arabinose centrifugation mother liquor were separate from the material. The solid arabinose was dried in operation 58 and the arabinose centrifugation mother liquor was recycled back for use in dissolution in operation 51 through the pipeline.

Operation 58, drying and packaging: a drying operation was performed on the solid arabinose obtained in operation 57 with 80°C hot air to obtain an arabinose crystal. The moisture content was controlled in a range of 0.15%-0.3%, and after the moisture content was in the range of 0.15%-0.3%, the arabinose crystal was cooled to 20°C-24°C using clean cold air at 12°C-15°C. The arabinose crystal obtained after drying was packaged using a packaging machine.

**Table 2. Comparison of key operational parameters and product yields for the embodiments and the control group.**

| Group | Operation | | | Arabinose content (%) | | Product yield (%) |
|---|---|---|---|---|---|---|
| | Ion exchange pH | Decolorization temperature (°C) | Evaporation temperature (°C) | Blending | Final product | |
| First Embodiment | 6.5 | 60 | 68 | 93.84% | 99.85% | 96.83 |
| Second Embodiment | 6.0 | 62 | 68 | 94.57% | 99.82% | 96.32 |
| Third Embodiment | 5.5 | 65 | 70 | 94.50% | 99.81% | 96.98 |
| Fourth Embodiment | 6.2 | 60 | 65 | 94..43% | 99.92% | 96.19 |
| Control group | 3.5∼4.0 | 75∼80 | 65∼98 | / | 99.27 | 92.93 |

By referring to Table 2, the arabinose content in the arabinose crystal prepared in each embodiment is all above 99.8%, and the product yield exceeds 96%. These results obtained according to the method of preparing the high-purity arabinose crystal in the present disclosure are superior to the arabinose crystal prepared in the control group, achieving the expected outcomes.

The above-described embodiments are merely the preferred embodiments of the present disclosure and should not be used to limit the scope of the present disclosure. Any modifications, equivalent substitutions, improvements, etc., made within the spirit and principles of the present disclosure should be included within the scope of protection of the present disclosure.

## Claims

1. A method for preparing a high-purity arabinose crystal, comprising:
operation 1, dissolution: dissolving a low-purity arabinose crystal to obtain a dissolved sugar solution using a dissolution tank system, wherein the dissolution tank system is provided with a temperature sensor and an automatic regulating valve, a Siemens distributed control system (DCS) is used to monitor a temperature during the dissolution to ensure that a temperature of the dissolved sugar solution is in a range of 55°C-60°C, and an arabinose content in the low-purity arabinose crystal is 96%-97%;
operation 2, blending: directing the dissolved sugar solution through a pipeline into a blending tank, adding an arabinose centrifugation mother liquor from operation 8 to the blending tank, and obtaining a blended sugar solution by blending the dissolved sugar solution with the arabinose centrifugation mother liquor, wherein a pH value of the blended sugar solution is in a range of 4.3-5.0, a dry basis concentration is in a range of 50%-60%, and the arabinose content of the blended sugar solution is 94±0.5%;
operation 3, ion exchange: performing an ion exchange operation on the blended sugar solution to obtain an ion-exchanged sugar solution, wherein a material temperature is controlled at 45°C-50°C through a heat exchanger for ion exchange feed, and a pH value of the ion-exchanged sugar solution is in a range of 5.5-6.5;
operation 4, decolorization and filtration: directing the ion-exchanged sugar solution through a pipeline into a decolorization tank for decolorization and filtration to obtain a decolorized sugar solution, wherein a decolorization temperature for decolorization and filtration is controlled at 60°C-65°C, and a pH value for decolorization and filtration is in a range of 5.5-7.5;
operation 5, fine filtration: performing a fine filtration operation on the decolorized sugar solution using a fine filtration membrane with an aperture of 0.45µm to obtain a refined sugar solution, wherein a temperature for fine filtration is controlled at 50°C-65°C, and a pH value for fine filtration is in a range of 5.0-7.5;
operation 6, evaporation and concentration: directing the refined sugar solution into a mechanical vapor recompression (MVR) evaporator for evaporation and concentration to obtain a concentrated sugar solution, wherein a temperature for evaporation and concentration is at 65°C-70°C, and a pH value for evaporation and concentration is in a range of 5.0-7.5;
operation 7, crystallization: introducing the concentrated sugar solution into a vacuum sugar-cooking system for crystallization, wherein a temperature for crystallization is at 63-65°C, and a vacuum level for crystallization is in a range of 70 mbar-90 mbar;
operation 8, centrifugation: performing a centrifugal separation operation on a material obtained in operation 7 to separate a solid arabinose and an arabinose centrifugation mother liquor from the material, wherein the arabinose centrifugation mother liquor is directed through the pipeline into the blending tank for blending in operation 2 and an arabinose content in the arabinose centrifugation mother liquor is in a range of 89%-91%.
operation 9, drying: performing a drying operation on the solid arabinose with 80°C hot air to obtain the high-purity arabinose crystal, wherein an arabinose content is greater than 99.8%.

2. The method of claim 1, wherein in operation 3, an ion exchange column is used in the ion exchange operation, and a ratio of cationic resins to anionic resins in the ion exchange column is 7:10.

3. The method of claim 1, wherein in operation 4, the decolorization and filtration operation includes adding activated carbon to the decolorization tank at a ratio of 1.2-1.4 Kg/ton of the dry basis, stirring at 110rpm for 30-45 minutes for decolorization, and using a plate and frame filter to filter and remove the activated carbon.

4. The method of claim 1, wherein in operation 7, the crystallization operation includes performing a vacuum evaporation crystallization by adding arabinose crystal seeds of 300-400 mesh in a ratio of two per ten thousand of the dry basis when an oversaturation degree of the concentrated sugar solution reaches 1.01-1.02, a crystallization period is 8 hours, and a stirring speed is at 80rpm.

5. The method of claim 1, wherein in operation 8, the centrifugal separation operation further includes controlling a washing time at 10 seconds and maintaining a water temperature at 55°C-60°C.

6. The method of claim 1, wherein in operation 9, the drying operation further includes controlling a moisture content in a range of 0.15%-0.3%, and after the moisture content is in the range of 0.15%-0.3%, cooling the high-purity arabinose crystal to 20°C -24°C using clean cold air with a temperature in a range of 12°C-15°C.
